(19) Europäisches Patentamt

European Patent Office

Office européen des brevets

(11) **EP 4 119 126 A1**

(12) **EUROPEAN PATENT APPLICATION**

(43) Date of publication:
**18.01.2023 Bulletin 2023/03**

(21) Application number: **22183842.8**

(22) Date of filing: **08.07.2022**

(51) International Patent Classification (IPC):
**A61K 9/107** (2006.01)      **A61K 9/19** (2006.01)
**A61K 9/51** (2006.01)      **A61K 36/9066** (2006.01)
**A61K 9/00** (2006.01)

(52) Cooperative Patent Classification (CPC):
**A61K 9/1075; A61K 9/0053; A61K 9/5123; A61K 9/5169; A61K 9/5192; A61K 36/9066; A61K 9/19**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.07.2021   IN 202141031451**

(71) Applicant: **Zeroharm Sciences Private Limited Ramkoti, Hyderabad, Telangana 500001 (IN)**

(72) Inventors:
• **Ravikanti, Swetha**
  **500014 Telangana (IN)**
• **Chitrabhanu, Lakshmanan**
  **641038 Tamil Nadu (IN)**

(74) Representative: **Eisenführ Speiser Patentanwälte Rechtsanwälte PartGmbB Postfach 31 02 60 80102 München (DE)**

(54) **NANOFORMULATION WITH DIVERSE FUNCTIONAL MOLECULES FROM TURMERIC AND PROCESS FOR PREPARATION OF THE SAME**

(57)      The present invention relates to a nanoparticle or nanoformulation comprising two or more bioactive phytochemicals from turmeric. The nanoparticle or nanoformuiation preferably comprises curcumin or curcuminoids and water-soluble peptides comprising turmerin. The waster-soluble peptides comprising turmerin are preferably present in a water-soluble extract of turmeric which comprises turmerin. Methods for producing the nanoparticle or nanoformuiation are also disclosed.

EP 4 119 126 A1

**Description**

**FIELD OF INVENTION**

The present invention is in the field of herbal nanoformulations, comprising two or more phytochemicals from turmeric.

**BACKGROUND OF THE INVENTION**

Turmeric (*Curcuma longa*) has been used since ancient times as a condiment, preservative, flavouring and cooking agent and contains many bio active molecules that are responsible for the nutraceutical and therapeutic properties of turmeric.
The bioactive compounds of *C. longa* have antioxidant, antitumor, antimicrobial, insecticidal, larvicidal, insect repellent, anticancer, anti-inflammatory, anti-metastatic, anti-angio-genic, healing and gastro-protective properties.
Curcuminoids are the key ingredient of turmeric and include curcumin, demethoxycurcumin, and bisdemethoxycurcumin. The content of the curcuminoid complex in the turmeric rhizome is between 2.5 and 6% and the variation in content is due to variety, climate and cultivation methodologies.
Apart from curcumin, turmeric is also a source of a wide variety of phytochemicals, each having its own nutritive and medicinal value. Interestingly turmeric also contains water-soluble peptides and the most documented peptide is turmerin. This heat stable, noncyclic peptide constitutes 0.1% of the dry weight of turmeric and is insensitive to trypsin, pepsin, heat and ultra violet radiation. Turmerin could inhibit enzymes linked to type 2 diabetes and hence turmeric is good for diabetic patients. Turmerin is an efficient antioxidant, non-cytotoxic anti-mutagen, and protectant of DNA[i].
Even though turmeric has functional molecules of great health and medicinal significance, turmeric products fail to offer sufficient bioavailability in humans. The clinical development of curcumin has been limited by its virtual insolubility in water at acidic pH, rapid hydrolysis at alkaline pH, limited absorption, poor bioavailability, rapid metabolism, quick systemic elimination, poor pharmacokinetics, low stability and low penetration targeting efficacy. Because of its poor absorption efficiency, it is difficult for orally administered curcumin to attain blood levels sufficient to exert its bioactivities. For curcumin to exhibit its therapeutic effects in the human body, a person is required to swallow between 12 and 20 g of curcumin every day to achieve substantial concentration in the body after ingestion.
Numerous approaches have been used to improve the bioavailability of curcumin, including, the use of adjuvants like piperine, liposomal curcumin, curcumin nanoparticles, curcumin-phospholipid complex, etc.[ii]
Shoba et al., (1998)[iii] showed that the concomitant administration of piperine with curcumin resulted in an enhanced serum concentration, extent of absorption and bioavailability of curcumin in both rats and humans with no adverse effects.
Bhawana et al., (2011) prepared curcumin nanoparticles with higher aqueous solubility by spraying a solution of curcumin in dichloromethane into boiling water, drop-wise, at a flowrate of 0.2 ml/min under ultrasonic conditions. It was found that both the choice of solvent and maintaining the drop flow were important parameters in obtaining the desired nano-particles.
To enhance oral bioavailability of curcumin, Grill et al., (2014)[iv] formulated curcumin with naturally occurring UGT inhibitors (piperine, quercetin, tangeretin and silibinin) in a self-microemulsifying drug delivery system. The study indicates that of the four inhibitors examined in vivo, silibinin significantly improved Cmax and the overall bioavailability of curcumin.
Artiga-Artigas et al., (2018)[v] assesses the influence of three molecularly different surfactants- lecithin, tween 20 and sucrose palmitate on the stability of curcumin loaded nanoemulsions and evaluates their influence on curcumin encapsulation and stabilization of curcumin-loaded nanoemulsions. The study concludes that nanoemulsions with 2.0% w/w lecithin did not suffer destabilization phenomena during almost 86 days of experiment, whereas those containing Tween 20 or SMP at the same concentrations were destabilized after 5 days or along 24 h, respectively.
Jayaprakasha et al., (2016)[vi] discloses the nanoencapsulation of curcumin with whey protein in different ratios to improve bioavailability and enhance its ability to prevent colon cancer.
Li et al., (2014)[vii] discloses preparation of nanoemulsion for water-insoluble curcumin stabilized by whey protein isolate. The results showed that nanoemulsions prepared using only whey protein isolate exhibited good stability over various ionic strengths, thermal treatments and upon storage.
WO201414585A1 discloses method for improving bioavailability of curcumin using milk protein concentrate, particularly, by using casein micelles.
WO2007110422A2 relates to whey protein micelles for delivery of active agents.
WO2015175573 A1 discloses curcumin-peptide conjugates for increasing the bioavailability of curcumin. In an embodiment, the complex is a curcumin- whey protein isolate complex.
WO 2010010431 discloses curcuminoid compositions comprising curcumin or curcuminoids, a lipidic carrier system with an HLB between 3 and 14, and a pH buffer, which forms a self nanoemulsion dilution with water, gastric fluid, or intestinal fluid of globule size of less than 200 nm.

Most of the formulations available in the market are primarily curcumin based and provide only partial benefits of turmeric to the consumers. But the profile of bioactive molecules in turmeric is highly promising and holds immense human health promoting potential. Therefore, it would be prudent to develop advanced nanoformulations, using a range of multifunctional bio active molecules from turmeric (such as, curcuminoids and peptides), which show high bioavailability upon oral administration. These multifunctional molecules exert additional health benefits compared to a formulation that contains only curcuminoids.

## SUMMARY OF THE INVENTION

The present invention aims to develop encapsulated nanoparticles that have the goodness of curcuminoids and other bio actives from turmeric, such as peptides including turmerin. This is achieved by converting the functional molecules from turmeric into nanoparticles and encapsulating them using nanoglobulins in combination with surfactants/emulsifiers. Formulations containing nano sized, encapsulated functional molecules have the benefit of high bioavailability, which in the present invention has been further enhanced by incorporating plant alkaloid(s) and flavonoid(s) and volatiles in the process.

The present invention provides nanoformulations comprising two or more bioactive phytochemicals from turmeric and methods of producing the same. In particular, the present invention deals with nanoparticles and nanoformulations comprising functional molecules from turmeric, such as, curcuminoids and water-soluble peptides, including turmerin. The advanced nanoparticles and nanoformulations of the present invention provide health benefits from multiple biomolecules from turmeric while improving their bioavailability and ensuring stability of the formulations.

The present invention also relates to a novel process to prepare anionic nanoparticles and nano formulations according to the present invention using a cationic encapsulant.

## DESCRIPTION OF THE DRAWINGS

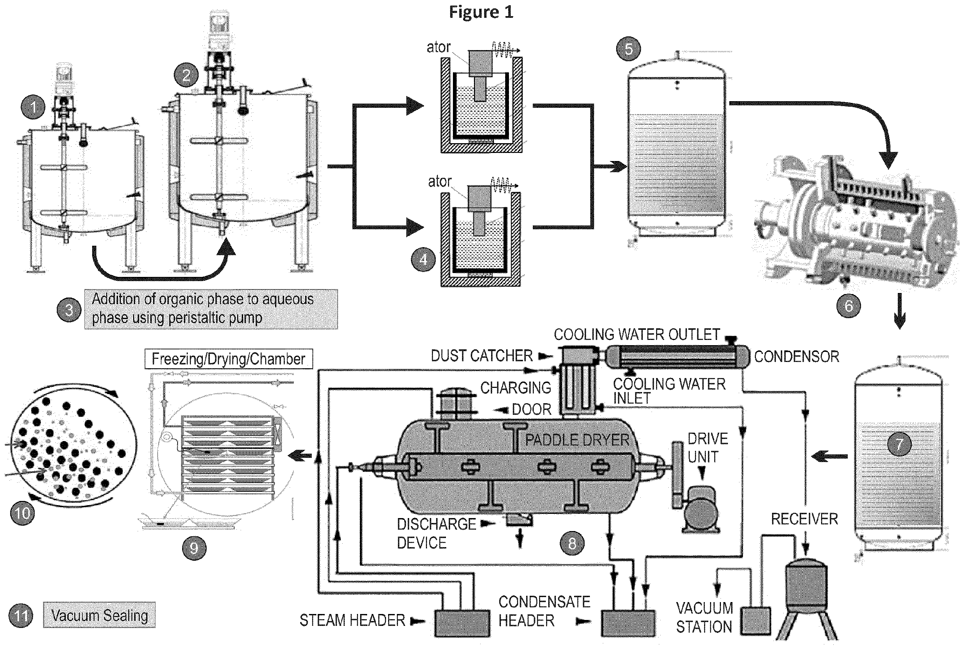

Figure 1 is a diagrammatic representation of the process involved in preparation of the multicomponent nanoparticles and nanoformulation of the present application, wherein (1) represents preparation of the organic phase (2) represents preparation of the aqueous phase (3) represents addition of organic phase into aqueous phase to obtain a coarse nanoemulsion (4) represents the division of the coarse nanoemulsion into two equal halves and subjecting the equal halves to ultrasonication (5) represents the pre-nanomilling buffering tank (6) represents size reduction of the nanoemulsion by wet grinding in a nanomiller (7) represents the post-nanomilling buffering tank (8) represents the volume reduction of the nanoemulsion using rotary vacuum paddle dryer (RVPD) (9) represents freeze drying of the nano emulsion to obtain dry powder (10) represents ball milling of the lyophilized powder to form a fine, free-flowing powder (11) represents vacuum sealing of the final product.

Figure 2 are graphs showing the particle size distribution of the multi-component nanoformulation of the present application (identified as 'nano curcumin' in the graph) and of unmodified curcuminoids (identified as 'curcumin' in the graph).

Figure 3 are graphs showing the zeta potential of the multi-component nanoformulation of the present application (identified as 'nano curcumin' in the graph) and of unmodified curcuminoids (identified as 'curcumin' in the graph).

Figure 4 shows the X-ray diffraction pattern of the multi-component nanoformulation of the present application (identified as 'nano curcumin' in the graph) and of unmodified curcuminoids (identified as 'curcumin' in the graph).

Figure 5a shows the ultraviolet visible spectra of unmodified curcuminoids.

Figure 5b shows the ultraviolet visible spectra of the multi-component nanoformulation of the present application.

Figure 6a shows the chemical structures of, (a) Curcumin, (b) Demethoxycurcumin and (c) Bis-demethoxycurcumin

Figure 6b is the FTIR Spectra comparing the nanoformulation of the present application (identified as 'nano curcumin' in the figure) and unmodified curcuminoid (identified as 'curcumin' in the graph).

Figure 6c shows the FTIR spectra of the unmodified aqueous turmeric extract (water-soluble extract comprising turmerin) in comparison with water-soluble extract comprising turmerin as present in the nanoformulations of the present application.

Figure 7 shows the standard calibration curve of analytical reagent (AR) grade curcuminoid (Sigma-Aldrich).

Figure 8a shows the release kinetics of curcuminoid from the the unmodified curcuminoid raw material (without encapsulation) in phosphate buffer (pH 7.4) that is fitted with zero order release. The unmodified curcuminoid shows burst release.

Figure 8b shows the release of curcuminoids from the nanoformulation in phosphate buffer (pH 7.4) when fitted with zero order release model.

Figure 8c shows the release of curcuminoids from the nanoformulation in phosphate buffer (pH 7.4) when fitted with Korsmeyer-Peppas model.

Figure 9 shows the effect of the nanoformulation of the present application (identified as 'nano formulated curcumin' in the figure) on the viability of SSC 25 cancer cell versus that of curcuminoid alone.

## DETAILED DESCRIPTION OF THE INVENTION

It is an object of the present invention to provide nanoparticles and nanoformulations comprising two or more bioactive phytochemicals from turmeric while achieving significant stability and bioavailability of the phytochemicals. The nanoparticles and nanoformulations of the present invention achieve size reduction, encapsulation and enrichment of the phytochemicals with plant alkaloids, which bring out the true potential of said phytochemicals. The process of the present application helps to achieve nanoparticles and nanoformulations with reduced size, encapsulation and enrichment of the phytochemicals with plant alkaloids, which bring out the true potential of said phytochemicals.

The present invention provides nanoparticles and nanoformulations comprising two or more bioactive phytochemicals from turmeric. Nanoparticles with specific nanometric size, surface charge and stability are described. The nanoparticles comprise bioactive molecules from turmeric along with one or more substances selected from plant derived-alkaloids, plant derived-flavonoids and volatiles, and are encapsulated using globulins.

The bioactive phytochemicals from turmeric that can be incorporated into the nanoparticles of the present application include curcumin, demethoxycurcumin, bisdemethoxycurcumin, turmerin, turmerones, turmeronols, etc.

In a preferred embodiment, the bioactive phytochemicals from turmeric are curcumin or curcuminoids and water-soluble peptides, including turmerin. The rhizomes of turmerin are rich in phenolic compounds, curcumin, demethoxycurcumin, and bisdemethoxycurcumin, that are together known as curcuminoid. Most of the commercial curcuminoids, sold as "Curcumin" are mixtures of curcumin, demethoxycurcumin (DMC) and bisdemethoxycurcumin (BDMC).

The term water-soluble peptides, including turmerin as used in the present application is used to denote water-soluble peptides from turmeric, which include turmerin. Said water-soluble peptides are present in a water-soluble extract comprising turmerin, obtained from turmeric. Said water-soluble extract comprising turmerin can be obtained through several processes, including, for example, the process as described in the present application.

In a preferred embodiment of the invention, successful encapsulation of phytochemicals is achieved through the use of globulins from whey protein as encapsulant.

The surface active agents or surfactants are mono- or di- esters of saturated or unsaturated fatty acids comprising 6 to 24 carbon atoms. The surface active agents preferably have a hydrophilic- lipophilic balance (HLB) between 4 and 17.

In the context of the present application, the terms "surface active agents" and "surfactant" have been used interchangeably.

In preferred embodiments, the surfactant is selected from the group consisting of polyoxyethylene sorbitan oleate (Tween 80), polyoxyethylene sorbitan stearate (Tween 60), polyoxyethylene sorbitan palmitate (Tween 60), polyoxyethylene sorbitan laurate (Tween 20), sorbitan laurate (Span 20), sorbitan palmitate (Span 40), sorbitan stearate (Span 60), sorbitan oleate (Span 20), Cremophor RH 40 and mixtures thereof.

The interfacial activity of surface active agents used plays a critical role in the characteristics and stability of the nanoparticles and nanoformulations. Stability is achieved by altering the composition of the surface active agents (non-ionic lipophilic and hydrophilic surfactants) and a combination of hydrophilic and lipophilic surfactants with HLB values between 4 and 17 are used. In a preferred embodiment, nonionic lipophilic and hydrophilic surfactants of HLB values 15 and 4.3 are used to bring the HLB value of the formulation to a desired level. In more preferred embodiments, the surfactants of choice are polyoxyethylene sorbitan oleate (Tween 80) and sorbitan stearate (Span 60).

In a preferred embodiment, the nanoparticle and nanoformulation are anionic.

The choice of surface active agents is critical in preventing the aggregation of nano droplets into larger ones and the concentration of span, tween and whey protein plays an important role in preventing droplet growth and its gravitational separation.

The concentration and ratio of span, tween and whey protein are designed for higher formulation stability by preventing droplet growth and gravitational separation. Small portions of plant derived alkaloids and flavonoids (bioenhancers) like quercetin, capsaicin, naringin, piperine and resveratrol and biopolymers are added alone or in combination to overcome poor membrane permeation and pre-systemic metabolic degradation of bioactive molecules from turmeric. The type and concentration of bioenhancers is critical in enhancing bioavailability of the turmeric bioactives.

In a preferred embodiment, the plant derived alkaloid is capsaicin and/or piperine. In a further preferred embodiment, the plant derived flavonoid is quercetin. The concentration of plant based bioenhancers, such as, quercetin, capsaicin and piperine that enhances the bioavailability of phytochemicals, has also been identified.

In a preferred embodiment, the HLB value of the nanoparticle or nanoformulation is around 10.

The nanoparticle or nanoformulation of the present application can be used in the treatment of various diseases like cancer.

Embodiments of the present application also deal with pharmaceutical compositions comprising a nanoparticle or nanoformulation of the present application and a pharmaceutically acceptable carrier.

The present invention also relates to a novel process to produce anionic nanoparticles and formulations using a cationic encapsulant. The present invention discloses a combined process (methodology) using ultra sonication, nano wet milling and dry ball milling for converting the functional phytochemicals into the nano size with low-crystallinity.

In an embodiment, the process of the present application comprises:

i. Preparation of an organic phase using ethyl acetate, a surface active agent with HLB 4.7 and curcuminoids.

ii. Preparation of an aqueous phase using water, a surface active agent with HLB 15, whey protein concentrate, water soluble extract, which comprises turmerin, and bioenhancers.

iii. Mixing the organic phase of step (i) with the aqueous phase of step (ii) in a defined ratio.

In a preferred embodiment the process of the present applications comprises:

i. Preparation of an organic phase by:

a. mixing ethyl acetate and sorbitan stearate (Span 60) until a clear homogenous solution is obtained

b. adding curcuminoids to the mixture of step i.a. and mixing until a clear homogenous solution is obtained.

ii. Preparation of an aqueous phase by:

a. Mixing water and polyoxyethylene sorbitan oleate (Tween 80) until a milky solution is obtained

b. Adding whey protein concentrate and water-soluble extract, which comprises turmerin to the solution of step ii.a. and mixing until a yellow milky solution is obtained.

c. Adding capsaicin and piperine to the solution of step ii.b.

iii. Mixing the organic phase of step (i) with the aqueous phase of step (ii) in a defined ratio of 1:4.
iv. Subjecting the emulsion of step iii. to ultrasonication.
v. Processing of nanoemulsion from step iv. using wet-grinding in a nano miller for size reduction.
vi. Volume reduction of nano-emulsion through solvent evaporation using rotary vacuum paddle dryer (RVPD).
vii. Freeze drying of slurry from rotary vacuum paddle dryer.
viii. Processing of freeze dried powder under ball miller to form fine free flowing powder.
ix. Vacuum sealing of processed freeze dried powder.

The ratio of the organic and aqueous phase is also critical for successful encapsulation. In a preferred embodiment, the ratio of the organic and aqueous phase is 1:4.

The encapsulant and surfactants have been selected and the process has been designed such that the signature functional groups of the phytochemicals from turmeric, are not affected as a result of the encapsulation.

In a preferred embodiment, the present invention relates to nanoparticles and nanoformulations of curcuminoids and water-soluble peptides. In a more preferred embodiment, the present invention relates to nanoparticles and nanoformulations of curcuminoids and water-soluble peptides, including turmerin. In a still more preferred embodiment, the present invention relates to nanoparticles and nanoformulations of curcumin and water-soluble peptides, including turmerin. In a preferred embodiment the nanoformulations of the present application are nanoemulsions. The nanoformulations of the present application can be administered orally, as a liquid or as tablets. The nanoformulations may also be encapsulated in a soft or hard gelatin capsule.

In a preferred embodiment, the multicomponent nanoparticles of the present application have a size range between 25-100 nm with an average size of 51 nm.

In a further preferred embodiment of the present invention, the multicomponent nanoparticles exhibit an anionic surface charge around neutral pH (pH 7).

In another embodiment, the present invention discloses a method of extracting a water-soluble extract from turmeric which comprises turmerin. The method comprises preparing an aqueous solution of turmeric by dissolving turmeric in water in a 1:8 (w/v) ratio. The resulting solution is subjected to ultrasonication. The ultrasonicated solution is then either subjected to centrifugation or filtration. The supernatant or filtrate is collected and the solvent is removed from the supernatant or filtrate using either a rotary evaporator or rotary vacuum paddle dryer. The sample is then subjected to lyophilization.

Unmodified curcuminoids/curcumin in the context of the present application refers to curcuminoids/curcumin in their raw state which have not been reduced to nanometric size.

Multi-component nanoparticles, nanoparticles and nanoformulations in the context of the present invention refer to nanoparticles or nanoformulations which comprise more than one functional molecule from turmeric.

The nano-particles and nanoformulations of the present application have the following composition:

| Component | Concentration |
|---|---|
| Curcuminoids | 0.30- 0.40 % w/v |
| Water-soluble extract comprising turmerin | 0.5-0.8% w/v |
| Span 60 | 0.3-0.5% w/v |
| Tween 80 | 0.4-0.6% w/v |
| Whey protein | 0.35- 0.5% w/v |
| Capsaicin | 0.004%w/v |
| Piperine | 0.005% w/v |
| Ethyl acetate | 20-30% v/v |
| Water | To make up to 100% |

The following examples are for the purpose of illustration of the invention and are not intended in any way to limit the scope of the invention.

**EXAMPLES**

**Example 1: Procedure for preparing multicomponent nanoformulation**

**Preparing the organic phase**

200 ml of ethyl acetate was taken in a jacketed vessel (1) with cooling facility. 4g of sorbitan stearate (Span 60) was added to the vessel containing ethyl acetate and mixed using high shear disperser until a clear, homogenous solution was obtained. Further 3.8g of curcuminoids were added to the solution and mixed thoroughly until a clear yellow solution was obtained. The curcumunoids used in the present application were procured from Dodla Natural Products, Chennai, India.

**Preparing the aqueous phase**

800 ml of demineralized water was taken in a jacketed vessel (2) and 6g of polyoxyethylene sorbitan oleate (Tween 80) was added to it and mixed vigorously using a high shear disperser to form a milky solution. The solution was milky white in color owing to the presence of the surfactant.

5g of whey protein concentrate and 8g of water-soluble extract, which comprises turmerin, were added upon thorough dissolution of the surfactant, and the solution was mixed well until a yellow, milky solution was obtained following which, the bioenhancers, capsaicin and piperine were added at 1% and 1.3%, to the weight of the curcuminoids, respectively.

The water-soluble extract comprising turmerin was obtained by extraction from fresh turmeric powder by utilizing a green solvent and ultrasound assisted extraction (UAE). Ultrasound-assisted extraction works on the principle of acoustic or ultrasonic cavitation and hydrodynamic shear force. When cavitation bubbles implode on the surface of plant cells/tissues, inter particle collisions causes surface peeling, erosion, and particle breakdown. The intercellular materials are transferred into the solvent because of the mechanical effects of ultrasound-induced cavitation. These mechanical effects also enhance the mass transfer of bioactives between the cells and solvent.

To generate the water-soluble extract for use in the nanoformulations of the present application, 1 g of commercially available turmeric powder was added to 8 ml of distilled water (1:8 ratio of turmeric powder and distilled water) and mixed thoroughly. The resulting solution was subjected to ultrasonication for 30 minutes. Ultrasonication was conducted using the following parameters:

1500 watt, 20 KHz
Amplitude ~ 50-60%
Temperature ~ 20°C-25°C
Pulse on ~ 10 sec, Pulse off ~5sec
Time ~ 30 mins

The extracted material was centrifuged at 10000 rpm for about 15 minutes. The supernatant was collected and transferred to a pear-shaped evaporating flask and the solvent was removed from the supernatant using a rotary evaporator at 65 °C for 1 hour. The samples were stored overnight at -80°C and were then subjected to lyophilization under the following conditions.

Freeze Drier Temperature ~ -50°C to -40°C
Vacuum pressure ~ 0.1 Pa
Time ~ 40 hours

In another experiment, the water-soluble extract for use in the nanoformulation of the present application, a 1:8 (w/v) solution of turmeric powder in distilled water (10Kg turmeric in 80L of distilled water) was subjected to ultrasonication for 120 minutes. The solution was then subjected to filtration using a sparkler filter or any other membrane filtration apparatus. The filtrate collected was placed in a Rotary Vacuum Paddle Dryer (RVPD) for removing the solvent. The RVPD was set for use at a vacuum of 560mm of Hg, a temperature of 55°C and the hot water tank temperature of 70°C. Finally, the material collected from RVPD was subjected to lyophilization.

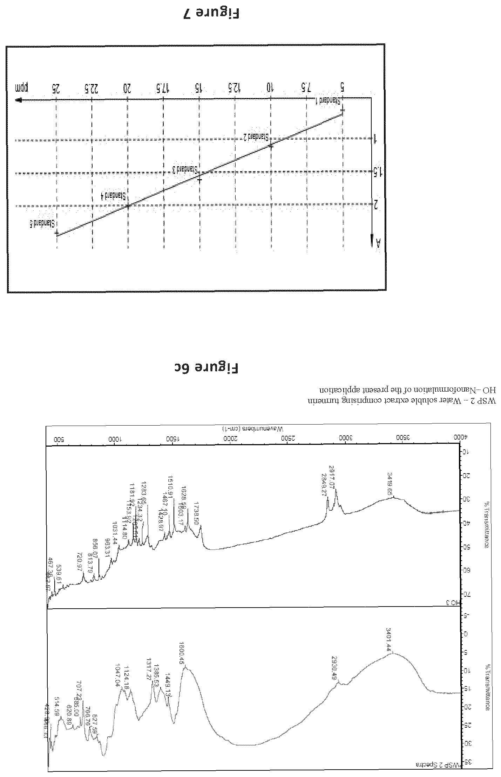

The lyophilized water-soluble extracts gave spectral peaks at 3401 cm$^{-1}$, 2930 cm$^{-1}$, 1124 cm$^{-1}$, 1047 cm$^{-1}$ and 685 cm$^{-1}$ as shown in Figure 6c (WSP 2 spectra).

**Preparing the nanoemulsion**

A coarse nanoemulsion was prepared by adding 200 ml organic phase into 800 ml aqueous phase with continuous mixing. The organic phase can be added to the aqueous phase using a peristaltic pump. The total emulsion produced was subjected to ultrasonication in a jacketed vessel (4).

The nanoemulsion was further processed by wet grinding in a nanomiller (6) for size reduction. The nanoemulsion was subjected to two cycles of wet grinding for 20 minutes followed by 10 minutes. The nanoemulsion can be placed in a buffering tank both prior to and after nanomilling (5, 7). The volume of the nanoemulsion was reduced using Rotary Vacuum Paddle Dryer (RVPD) (8) operated at a temperature of 63°C and vacuum of 650 mTorr. The slurry obtained using rotary vacuum paddle dryer was freeze dried (9) under the following temperature and vacuum cycle:

Temperature: -35 to + 30 in 12 hours cycle
Vacuum cycle: 300 to 400 mTorr in 12 hours cycle

A powder form of the nanoemulsion was obtained using freeze-drying. The freeze-dried powder was processed in a ball miller (10) to form a fine, free-flowing powder which was then vacuum sealed (11).

**Table 1:** The following is representative of a nanoformulation of the present application:

| Component | Concentration |
|---|---|
| Curcuminoids | 0.40 % w/v |
| Water-soluble extract which comprises turmerin | 0.8% w/v |
| Span 60 | 0.4% w/v |
| Tween 80 | 0.6% w/v |
| Whey protein | 0.5% w/v |
| Capsaicin | 0.004%w/v |
| Piperine | 0.005% w/v |
| Ethyl acetate | 20% v/v |
| Water | To make up to 100% |

The present nanoformulation has been designed to achieve an HLB value of around 10.0, which is desirable for an oil-in-water nano-emulsion.

**Example 2: Characterization of curcuminoids and the multicomponent nanoparticles of the present application**

**Example 2A: Particle size Analysis**

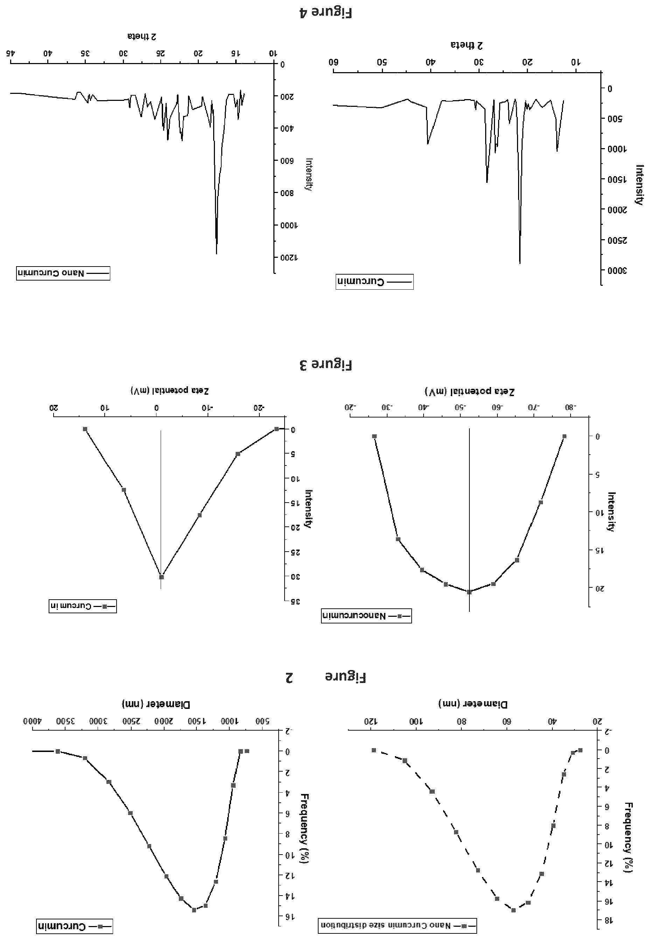

The size of the samples was analyzed using Nano Particle Size Analyzer (Model: HORIBA-SZ-100). The size was measured based on the principle of dynamic light scattering. Figure 2 shows that the particle size distribution of unmodified curcuminoids was between the size range of 1000-2000 nm whereas the multicomponent nanoparticles of the present application were distributed over the size range of 25-100 nm with an average size of 51 nm.

The Polydispersity Index (PI) is a measure of heterogeneity in a sample based on size and PI values of less than 1 are preferred. The PI value obtained for the multicomponent nanoformulation of the present application was 0.425 indicating that the size distribution is homogenous.

**Example 2B: Surface Charge**

Zeta Potential is the potential difference between the dispersion medium and the stationary layer of fluid attached to the particles. It is a characterization technique for estimating the surface charge of the particles and is used for attaining the right surface charge that can ensure physical stability of the nanosuspensions. The multicomponent nanoformulations of the present application were stable with a zeta potential of -49mV while the zeta potential of unmodified curcuminoids was >-10 mV (Fig 3). The positive and negative charge of the zeta potential is directly proportional to the surface charge of the sample. The negative charge obtained for the multicomponent nanoformulations of the present application indicates that both unmodified curcuminoids and the multicomponent nanoformulations and nanoparticles of the present application are anionic in nature.

Even though the encapsulant (whey protein) used is positively charged, the anionic nature of the formulation is due to the influence of pH. The surface charge of whey protein in acidic pH is positive, while under alkaline pH it is negatively charged. As the nanoformulation of the present invention is around neutral pH, it is anionic in nature.

**Example 2C: X-ray diffraction**

X-ray diffraction (XRD) is a technique used for analyzing the atomic or molecular structure of materials and in predicting the crystallinity of a sample. The health benefits of curcumin have not yet been realized due to its low bioavailability (~1%) which in turn is due to its low water solubility, chemical instability and crystalline structure. Thus, the main objective of the present novel nanoformulation was to achieve low-crystallinity which can result in improved bio availability. The XRD patterns of unmodified curcuminoid powder exhibit a series of thin and intense lines, which are indicative of crystalline nature of the material. While the nanoparticles of the present application have similar peaks, the intensity of crystallinity is found to have been reduced (Fig. 4).

**Example 2D: Ultraviolet Visible Spectra**

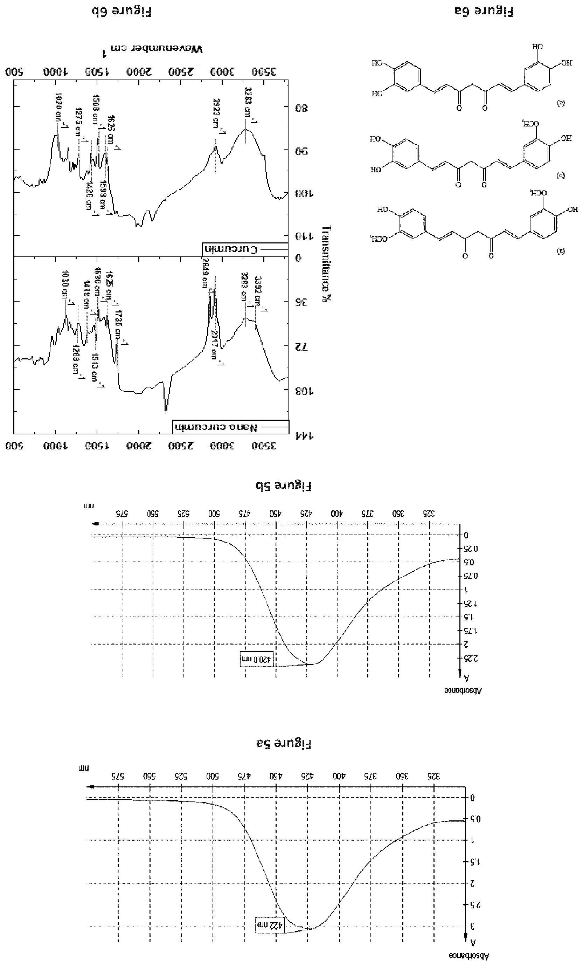

Spectrometers can accurately distinguish and quantify radiation in the ultraviolet, visible, and near infrared regions of the spectrum. They work based on calorimetric principle and the absorbance of curcumin (yellow pigment) is usually observed between 420 nm to 435 nm. It is apparent from the results of ultraviolet visible spectrometry shown in Fig 5a and Fig 5b that the absorbance peak at 422 nm and 420 nm and the absorbance intensity in the multicomponent nanoparticle of the present application has not been disturbed relative to unmodified curcuminoids. This shows that the nature of the curcuminoids in the multi-component nanoparticles of the present application has not been disturbed.

**Example 2E: Fourier Transform Infra-Red Spectroscopy (FTIR Spectroscopy)**

**FTIR Spectroscopy characterization of curcuminoids**

The main compounds contributing to the activities of C. *longa* Linn are curcuminoids, including curcumin, demethoxycurcumin and bis-demethoxycurcumin.

The infrared spectroscopy is measured between 4000 $cm^{-1}$ to 400 $cm^{-1}$. Infra-red active molecules have unique IR absorption spectra that may be used as a "fingerprint" for identification. Since each functional group absorbs a specific wavelength, no two unique molecules have the same absorption spectra. Thus, this technique is utilized to identify the functional groups.

The major functional groups in curcumin are an $\alpha$, $\beta$-unsaturated $\beta$-diketone moiety and an aromatic O-methoxy-phenolic group. The aromatic ring systems, which are phenols, are connected by two $\alpha$, $\beta$-unsaturated carbonyl groups. The FTIR spectrum obtained confirms the presence of signature peaks in curcumin.

The FTIR spectra of unmodified curcuminoid samples and the multicomponent nanoparticles of the present application are provided in figures 6a and 6b.

The peak at 3283 $cm^{-1}$ in both unmodified curcuminoid samples and the multicomponent nanoparticles of the present application corresponds to the phenolic O-H stretching vibration.

The bands at 2849 $cm^{-1}$ observed in the multicomponent nanoparticles of the present application correspond to $-CH_2$ group in the whey protein concentrate, that was used for encapsulation.

The peak at 1626 $cm^{-1}$ in unmodified curcuminoid sample pertaining to the aromatic moiety C=C stretching has a mild shift at 1625 $cm^{-1}$ in the multicomponent nanoparticles of the present application.

The benzene ring stretching vibrations in unmodified curcuminoid sample is at 1598 $cm^{-1}$, whereas the same is shifted to 1580 $cm^{-1}$ in the multicomponent nanoparticles of the present application. The shift is due to the bending of the benzene ring and the primary amine group of whey protein concentrate.

The peak, 1508 $cm^{-1}$ in unmodified curcuminoid sample corresponds to the C=O vibrations whereas the same has a slight shift and is observed at 1513 $cm^{-1}$. As the C=O bond overlaps the secondary amide group of protein in whey protein.

1428 $cm^{-1}$ corresponds to olefinic C-H bending vibrations in unmodified curcuminoid sample which, in the multicomponent nanoparticles of the present application is at 1419 $cm^{-1}$ with a minor shift that is due to the blending of C-C in whey protein concentrate and C-H bonding in curcumin.

1275 $cm^{-1}$ in unmodified curcuminoid sample and 1268 $cm^{-1}$ in the multicomponent nano-particles of the present application corresponds to the aromatic C-O stretching vibrations respectively.

1020 $cm^{-1}$ and 1030 $cm^{-1}$ correspond to C-O-C stretching vibrations in unmodified curcuminoid sample and the multicomponent nanoparticles of the present application, respectively.

The similar spectral peaks in the multicomponent nanoformulations of the present application evidence that the signature functional groups of curcumin were not affected by encapsulation in the nanoparticles of the present application. The peaks at 1580 $cm^{-1}$ and 1513 $cm^{-1}$ show the interaction between the active compound, curcumin and the encapsulant, whey protein concentrate in the multicomponent nanoparticles and nanoformulations of the present application.

**FTIR Spectroscopy characterization of water-soluble extract comprising turmerin**

The FTIR spectra of the unmodified water-soluble extract comprising turmerin in comparison with the water-soluble extract comprising turmerin as present in the nanoformulations of the present application is shown in Figure 6c.

In the unmodified water-soluble extract comprising turmerin, the stretching vibration of O-H creates a broad band at 3401 $cm^{-1}$, showing the presence of flavonoids and other phenolic compounds. The specific band at 2930 $cm^{-1}$ corresponds to aliphatic C-H stretching vibration. This band might represent glycosides, which are several forms of sugar molecules. The spectral peaks observed at 1124 $cm^{-1}$ and 1047 $cm^{-1}$ represents C-O stretching. The peak at 685 $cm^{-1}$ probably due to the CH (OOP) bending. (Figure 6C, WSP 2 spectra)

When the water-soluble extract comprising turmerin is impregnated in the nanoformulations, the peak locations of turmerin and shape showed a shift similar to that seen with the curcuminoids. The peaks of turmerin were shifted from 3401 cm$^{-1}$ to 3419 cm$^{-1}$, 1124 cm$^{-1}$ to 1153 cm$^{-1}$ and 1047 cm$^{-1}$ to 1114 cm$^{-1}$ after encapsulation into the nanoformulations of the present application.(Figure 6c, HO 3)

In this characterization experiment, the nanoformulations of the present application also show spectral peaks representative of curcuminoids at 1628 cm$^{-1}$ which represents aromatic moiety C=C stretching, a peak at 1603 cm$^{-1}$ which is due to the benzene ring stretching vibrations), a sharp peak at 1510 cm$^{-1}$ which indicates the presence of C=O and C=C vibrations, 1428 cm$^{-1}$ which corresponds to olefin C-H bending vibrations and C-O-C stretching vibrations that generate the peak at 1024 cm$^{-1}$. The intense peaks at 2917 cm$^{-1}$ and 2849 cm$^{-1}$ represent the phospholipids due to CH2 stretching vibration. (Figure 6c, HO 3)

**Example 3: Entrapment Efficiency of Curcuminoids in the Nanoformulations**

**Example 3A. Standard Absorbance Graph of Curcuminoids**

Using analytical reagent (AR) grade curcuminoid (Sigma-Aldrich) as a blank, 200 ppm of curcuminoid solution was scanned in the UV-Visible spectroscopy range of 200 to 1100 nm. The maximum absorbance of curcuminoid was found at 404 nm.

The standard calibration curve of curcuminoids (Figure 7) was prepared by plotting the values of absorbance of stock solutions of 5, 10, 15, 20 and 25 ppm of AR grade curcuminoid, observed at 404 nm with the regression coefficient, $r^2$ value of 0.99.

| | |
|---|---|
| R2 adjust (Calculated/Default) | 0.9900 / 0.9900 |
| Wavelength [nm] | 404 |
| Concentration Min. | 5.00 ppm |
| Concentration Max. | 25.00 ppm |
| Cell Path length [cm] | 1.0000 |

**Table 2: Absorbance of curcuminoid stock solutions of different concentrations at 404 nm**

| Curcuminoid concentration (ppm) | Absorbance at 404nm |
|---|---|
| 5 | 0.5699 |
| 10 | 1.1153 |
| 15 | 1.6131 |
| 20 | 2.0109 |
| 25 | 2.4203 |

**Example 3B. Entrapment Efficiency of in the Nanoformulations**

UV- Visible spectroscopy at an absorbance of 404 nm was used for evaluation of entrapment efficiency of curcuminoids in the nanoparticles or nanoformulations of the present application. The absorbance was measured at 404 nm after dilution and centrifugation with acetone.

1 mL of the nanoformulation of the present application was diluted with 9 mL of acetone and centrifuged for 30 minutes at 10000 rpm. The supernatant was then filtered through a 0.45 micron filter, and the absorbance of the filtrate was measured at 404 nm with acetone as a blank.

The concentration of curcuminoids was determined using the absorption data and the standard calibration curve of curcuminoids. Entrapment Efficiency (EE) was calculated according to the following formula:

EE(%) =

Total amount of curcuminoids in the nanoformulation- Amount of curcuminoids in the filtrate X 100

Total amount of curcuminoids in the nanoformulation

The entrapment efficiency of curcuminoids in the multicomponent nanoparticles or nanoformulations of the present application was found to be 71.20%. The results showed that 178 ppm of curcuminoid was loaded in the nanoparticles and nanoformulations of the present application

**Example 4: *In vitro* Release Kinetics**

*In vitro* release kinetics studies were carried out in accordance with the protocol of Asghar *et al.,* 2017[viii] with minor modifications. 10 ml of the present nanoformulation was transferred to a dialysis bag comprising dialysis membrane 60 (molecular weight cut off 12000 to 14000 KDa). The bag was sealed on both sides before being placed in a 1000 ml beaker containing 500 ml phosphate buffer (pH7.4) and was continuously stirred at 50 rpm. 3 ml of the release medium was withdrawn at regular intervals of 3 hours for 48 hours (0, 3, 6, 9, 12, 15, 18, 21, 24, 27, 30, 33, 36, 39, 42, 45, 48 hours) and equal amount of fresh buffer was added to maintain the sink conditions each time the release medium was withdrawn. The samples were analyzed using UV-Visible Spectroscopy at an absorbance of 404 nm.

The release data were fitted to zero order, first order and Korsmeyer-Peppas models to evaluate release mechanisms and kinetics. The results confirmed the controlled release of curcuminoids from the final nanoformulation. Controlled and sustained release of the active molecule is a prerequisite for higher bioavailability. In phosphate buffer, the release of curcuminoids was 25%, 49 % and 82 % at 12 hours, 24 hours and 48 hours, respectively.

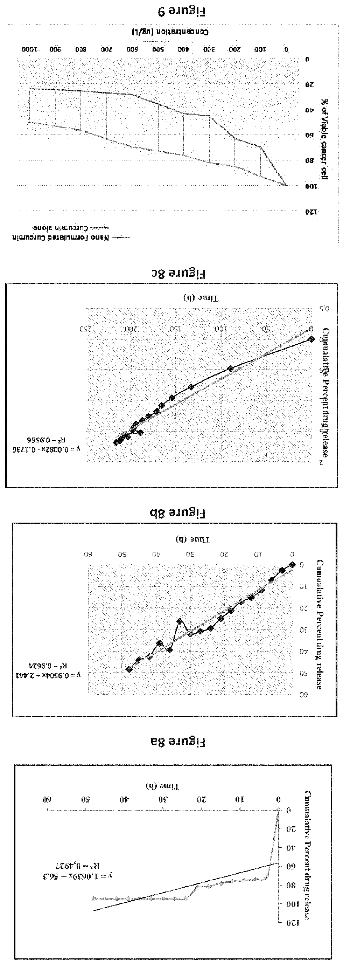

Figure 8a shows the release kinetics of curcuminoid from the the curcuminoid raw material (without encapsulation in the present nanoformulation) in phosphate buffer (pH 7.4) that is fitted with zero order release. The raw curcuminoid shows burst release.

The release of curcuminoid from the nanoformulation in phosphate buffer (pH 7.4) was fitted with zero order release model with $R^2$ value of 0.96 (Figure 8b) and the Korsmeyer-peppas model with $R^2$ value of 0.956 (Figure 8c). The release kinetics of curcumin and its complex in both simulated gastrointestinal and simulated saliva conditions followed a Fickian diffusion mechanism. The release data were well fitted with zero order and Korsmeyer-Peppas models with $R^2$ values higher than 0.9176 (Rezaei & Nasirpour, 2019[ix]). Encapsulation using whey protein slowed the release of curcuminoids in the simulated gastrointestinal (GI) environment.

**Table 3: In vitro Release Kinetics of curcuminoids from the Nanoformulations of the present application versus that of unmodified curcuminoids**

| S.No | Release Kinetics Model | $R^2$ of Unmodified Curcuminoids | $R^2$ of Curcuminoids from Nanoformulation |
|---|---|---|---|
| 1 | Zero order | 0.493 | 0.962 |
| 2 | First order | 0.228 | 0.648 |
| 3 | Korsmeyar Peppas Model | 0.556 | 0.956 |

From the foregoing table it is apparent that while unmodified curcuminoids show burst release, while the nanoformulations of the present application show controlled release of curcuminoids.

**Example 5: Efficacy of Curcuminoids in the Multicomponent Nanoparticles of the Present Application in Comparison with Curcuminoids (alone) on SSC-25 Cell Lines**

Cell line studies were carried out on SSC-25 cell line to confirm the efficacy of nanoformulated curcuminoids on proliferation and invasion of said cells. The Squamous Cell Carcinoma Cell lines "SSC-25" was obtained from ATCC (American Type Culture Collection), USA.

Oral Squamous Cell Carcinoma (OSCC) is the most frequent malignant tumour in the oral cavity and the survival rate is less than 50%.

The expression of Epidermal Growth Factor Receptor (EGFR) is increased during OSCC and is associated with tumor growth, invasion and metastasis. There are number of studies reported on the effect of curcumin on EGFR downstream signalling pathways. But the nanoformulation of the present application was found to have a greater impact on down-regulation of EGFR.

The cells were treated with different concentrations of curcuminoid alone or the nanoformulation of the present application [(100 $\mu$g/L to 1000 $\mu$g /L) for 25 and 48 hours. The proliferation of SCC-25 cells was detected by MTT assay.

A dose dependent inhibition of cell proliferation was observed. An increased rate of inhibition of cancer cell proliferation was seen with increasing concentration of the nano-formulated curcuminoid. It can be seen from figure 9 that the nanoformulation of the present application (referred to as "Nano Formulated Curcumin" in the figure) had a greater impact on reducing cancer cell viability as compared to curcuminoid alone (referred to as "Curcumin alone" in the figure).

As the nanoformulation had better effect on inhibition of SSC-25 cell proliferation, apoptosis studies were also conducted to prove the effect of the nanoformulation according to the present invention, on proliferation of healthy cells. No cell death was observed in healthy cells. Thus, the nanoformulation according to the present invention is safe to healthy cells.

Cell invasion is the spread of cancer cell to neighbouring cells, tissues and organs that may lead to secondary and tertiary cancer which accounts for higher mortality. A cell invasion assay was also performed using the nanoformulation of the present application. The nanoformulated curcuminoid according to the present invention evidently reduced the cell invasion in comparison with curcuminoid (alone) formulation. One of the reasons behind the inhibition of cell invasion could be the decreased activity of extracellular matrix metal-loproteinases such as MMP-2 and MMP-9, which degrade most of extracellular matrix proteins during organogenesis and which play a key role in local tumor invasion.

[i] Leela Srinivas, V. EC. Shalini, and M. Shylaja, Turmerin: A Water Soluble Antioxidant Peptide from Turmeric [Curcuma longa], Archives of Biochemistry and Biophysics, Vol. 292, No. 2, February 1, pp. 617-623, 1992

[ii] Preetha Anand, Ajaikumar B. Kunnumakkara, Robert A. Newman and Bharat B. Aggarwal, Bioavailability of Curcumin: Problems and Promises, Mol. Pharmaceutics 2007, 4, 6, 807-818

[iii] G. Shoba, D. Joy, T. Joseph, M. Majeed, R. Rajendran, and P. S. Srinivas. Influence of piperine on the pharmacokinetics of curcumin in animals and human volunteers, Planta medica 64: 353-6 (1998)

[iv] Alex E. Grill & Brenda Koniar & Jayanth Panyam. Co-delivery of natural metabolic inhibitors in a self-microemulsifying drug delivery system for improved oral bioavailability of curcumin, Drug Deliv. and Trans1. Res., 7th May 2014

[v] Maria Artiga-Artigas, Yamel Lanjari-Perez, Olga Martín-Belloso. Curcumin-loaded nanoemulsions stability as affected by the nature and concentration of surfactant, Food Chemistry 266 (2018) 466-474

[vi] Guddadarangavvanahally K Jayaprakasha, Kotamballi N Chidambara Murthy and Bhimanagouda S Patil. Enhanced colon cancer chemoprevention of curcumin by nanoencapsulation with whey protein. European Journal of Pharmacology, Volume 789, 15 October 2016, Pages 291-300

[vii] Ming Li, Ying Ma, Jie Cui. Whey-protein-stabilized Nanoemulsions as a Potential Delivery System for Water-insoluble Curcumin, Food Science and Technology (2014) 1-10

[viii] Asghar, Khushnuma, Mohd Qasim, Gangappa Dharmapuri, and Dibakar Das. "Investigation on a smart nanocarrier with a mesoporous magnetic core and thermo-responsive shell for co-delivery of doxorubicin and curcumin: a new approach towards combination therapy of cancer." RSC advances 7, no. 46 (2017): 28802-28818.

[ix] Rezaei, Atefe, and Ali Nasirpour. "Evaluation of release kinetics and mechanisms of curcumin and curcumin-β-cyclodextrin inclusion complex incorporated in electrospun almond gum/PVA nanofibers in simulated saliva and simulated gastrointestinal conditions." BioNanoScience 9, no. 2 (2019): 438-445.

**Claims**

1. A nanoparticle or nanoformulation comprising two or more bioactive phytochemicals from turmeric, one or more surfactants, globulins, and one or more of substances selected from plant-derived alkaloids, plant-derived flavonoids and volatiles.

2. The nanoparticle or nanoformulation according to claim 1, wherein the two or more bioactive phytochemicals from turmeric are selected from curcumin, demethoxycurcumin, bisdemethoxycurcumin, turmerones, turmeronols and water-soluble peptides including turmerin.

3. The nanoparticle or nanoformulation according to claim 1, wherein the bioactive phytochemicals from turmeric are curcumin or curcuminoid and water-soluble peptides, including turmerin and/or wherein the water-soluble peptides, including turmerin are present in a water-soluble extract comprising turmerin obtained from turmeric.

4. The nanoparticle or nanoformulation according to claim 1, wherein the globulins are globulins from whey protein.

5. The nanoparticle or nanoformulation according to claim 1, wherein the surfactants are mono- or di- esters of saturated or unsaturated fatty acids comprising 6 to 24 carbon atoms, and/or wherein the surfactants have a hydrophilic-lipophilic balance (HLB) between 4 and 17, preferably wherein the surfactants are nonionic lipophilic and hydrophilic surfactants of HLB values 15 and 4.3.

6. The nanoparticle or nanoformulation according to claim 5, wherein the surfactant is selected from the group consisting of polyoxyethylene sorbitan oleate (Tween 80), polyoxyethylene sorbitan stearate (Tween 60), polyoxyethylene sorbitan palmitate (Tween 60), polyoxyethylene sorbitan laurate (Tween 20), sorbitan laurate (Span 20), sorbitan palmitate (Span 40), sorbitan stearate (Span 60), sorbitan oleate (Span 20), Cremophor RH 40 and mixtures thereof, preferably wherein the surfactants are polyoxyethylene sorbitan oleate (Tween 80) and sorbitan stearate (Span 60).

7. The nanoparticle or nanoformulation according to claim 1, wherein the plant-derived alkaloids and plant-derived flavonoids are selected from quercetin, capsaicin, naringin, piperine and resveratrol and mixtures thereof, preferably wherein the plant-derived alkaloids and the plant-derived flavonoid are selected from capsaicin, piperine, quercetin and mixtures thereof.

8. The nanoparticle or nanoformulation according to claim 1, comprising

    (i) curcumin or curcuminoids;
    (ii) water-soluble extract, comprising turmerin;
    (iii) polyoxyethylene sorbitan oleate (Tween 80);
    (iv) sorbitan stearate (Span 60);
    (v) capsaicin;
    (vi) piperine; and
    (vii) whey protein.

9. The nanoparticle or nanoformulation according to claim 8, comprising 0.30- 0.40 % w/v of curcuminoids, 0.5-0.8% w/v of water-soluble extract comprising turmerin, 0.3- 0.5% w/v of Span 60, 0.4-0.6% w/v of Tween 80, 0.35- 0.5% w/v of whey protein, 0.004% w/v of capsaicin and 0.005% w/v of piperine.

10. The nanoparticle or nanoformulation according to claim 9, comprising 0.4 % (w/v) curcuminoids, 0.8% (w/v) water-soluble extract comprising turmerin, 0.6% (w/v) polyoxyethylene sorbitan oleate (Tween 80); 0.4% (w/v) sorbitan stearate (Span 60); 0.004% (w/v) capsaicin, 0.005% (w/v) piperine and 0.5%% (w/v) whey protein.

11. The nanoparticle or nanoformulation according to any one of claims claim 8 to 10, further comprising 20-30% v/v of ethyl acetate and water to make up the volume to 100%, preferably further comprising 20% v/v ethyl acetate and water to make up the volume to 100%.

12. The nanoparticle or nanoformulation according to any of the preceding claims wherein the water soluble extract comprising turmerin is prepared by a method comprising:

    (i) Preparing an aqueous solution of turmeric by dissolving turmeric in water in a 1:8 (w/v) ratio.
    (ii) Subjecting the resulting solution to ultrasonication.
    (iii) Filtering or centrifuging the solution obtained after ultrasonication in step (ii).
    (iv) Collecting the filtrate or supernatant and removing the solvent using a rotary vacuum paddle dryer (RVPD) or rotary evaporator.
    (v) Lyophilizing the product of step (iv).

13. The nanoparticle or nanoformulation according to claim 1, wherein the nanoparticle or nanoformulation:

    (i) is anionic around neutral pH; and/or
    (ii) has an HLB value around 10; and/or
    (iii) has a size range of 25-100 nm with an average size of 51 nm; and/or
    (iv) is a nanoemulsion.

14. A pharmaceutical composition comprising a nanoparticle or nanoformulation according to any one of the preceding claims and a pharmaceutically acceptable carrier.

15. A process for preparing a nanoparticle or nanoformulation comprising:

    (i) Preparing an organic phase using ethyl acetate, a surfactant with HLB 4.7 and curcuminoids;
    (ii) Preparing an aqueous phase using water, a surfactant with HLB 15, whey protein concentrate, water soluble extract, which comprises turmerin, and bioenhancers; and
    (iii) Mixing the organic phase of step (i) with the aqueous phase of step (ii) in a defined ratio.

**16.** The process according to claim 15, wherein:

(i) the ratio of the organic and aqueous phase in step (iii) is 1:4; and/or
(ii) the surfactant with HLB 4.7 is sorbitan stearate (Span 60) and the surfactant with HLB 15 is polyoxyethylene sorbitan oleate (Tween 80); and/or
(iii) the bioenhancers are plant-derived alkaloids, preferably capsaicin and piperine.

**17.** A process for preparing a nanoparticle or nanoformulation comprising:

(i) Preparing an organic phase by:

a. mixing ethyl acetate and sorbitan stearate (Span 60) until a clear homogenous solution is obtained.
b. adding curcuminoids to the mixture of step (i)a. and mixing until a clear homogenous solution is obtained.

(ii) Preparing an aqueous phase by:

a. Mixing water and polyoxyethylene sorbitan oleate (Tween 80) until a milky solution is obtained
b. Adding whey protein concentrate and water soluble extract, which comprises turmerin to the solution of step (ii)a. and mixing until a yellow milky solution is obtained.
c. Adding capsaicin and piperine to the solution of step (ii)b.

(iii) Mixing the organic phase of step (i) with the aqueous phase of step (ii) in a defined ratio of 1:4.
(iv) Subjecting the emulsion of step iii. to ultrasonication.
(v) Processing of nanoemulsion from step iv. using wet-grinding in a nano miller for size reduction.
(vi) Volume reduction of nano-emulsion through solvent evaporation using rotary vacuum paddle dryer (RVPD).
(vii) Freeze drying of slurry from rotary vacuum paddle dryer.
(viii) Processing of freeze dried powder under ball miller to form fine free flowing powder.
(ix) Vacuum sealing of processed freeze dried powder.

**18.** The process according to any one of claims 16 to 17, wherein the water-soluble extract, comprising turmerin is prepared by a method comprising:

(i) Preparing an aqueous solution of turmeric by dissolving turmeric in water in a 1:8 (w/v) ratio.
(ii) Subjecting the resulting solution to ultrasonication.
(iii) Filtering or centrifuging the solution obtained after ultrasonication in step (ii).
(iv) Collecting the filtrate or supernatant and removing the solvent using a rotary vacuum paddle dryer (RVPD) or rotary evaporator.
(v) Lyophilizing the product of step (iv).

**19.** A nanoparticle or nanoformulation obtained by the process according to any one of claims 16 to 18.

Figure 1

Figure 2

Figure 3

Figure 4

Europäisches
Patentamt
European
Patent Office
Office européen
des brevets

# EUROPEAN SEARCH REPORT

**Application Number**

EP 22 18 3842

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GÓMEZ-MASCARAQUE LAURA G ET AL: "Microencapsulation structures based on protein-coated liposomes obtained through electrospraying for the stabilization and improved bioaccessibility of curcumin", FOOD CHEMISTRY, ELSEVIER LTD, NL, vol. 233, 22 April 2017 (2017-04-22), pages 343-350, XP085491860, ISSN: 0308-8146, DOI: 10.1016/J.FOODCHEM.2017.04.133 | 1,4,5,13 | INV. A61K9/107 A61K9/19 A61K9/51 A61K36/9066 A61K9/00 |
| Y | * abstract * * page 344, left-hand column, line 50 - right-hand column, line 8 * * page 344, right-hand column, lines 25-32 * ----- | 2,3,7 | |
| Y | AMALRAJ AUGUSTINE ET AL: "Preparation of a novel bioavailable curcuminoid formulation (Cureit(TM)) using Polar-Nonpolar-Sandwich (PNS) technology and its characterization and applications", MATERIALS SCIENCE AND ENGINEERING C, ELSEVIER SCIENCE S.A, CH, vol. 75, 15 February 2017 (2017-02-15), pages 359-367, XP029978089, ISSN: 0928-4931, DOI: 10.1016/J.MSEC.2017.02.068 * abstract * * page 362, left-hand column, lines 6-14 * ----- -/-- | 2,3 | TECHNICAL FIELDS SEARCHED (IPC) A61K |

The present search report has been drawn up for all claims

| Place of search | Date of completion of the search | Examiner |
|---|---|---|
| The Hague | 28 November 2022 | Zalfen, Alina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 22 18 3842

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | G SHOBA ET AL: "Influence of piperine on the pharmacokinetics of curcumin in animals and human volunteers", PLANTA MED., vol. 64, no. 4, 1 January 1998 (1998-01-01), pages 353-356, XP055218622, DOI: 10.1055/s-2006-957450 * abstract * | 7 | |
| A | CHITHDAVONE HER ET AL: "Improvement of Curcumin Bioavailability for Medical Applications", MEDICINAL & AROMATIC PLANTS, vol. 7, no. 6, 15 December 2018 (2018-12-15), pages 1-15, XP055744459, DOI: 10.4172/2167-0412.1000326 * abstract * | 1-19 | |
| A | US 2018/185282 A1 (GUERRERO RIVERA SIMÓN JUAN [CL] ET AL) 5 July 2018 (2018-07-05) * paragraph [0011] – paragraph [0012] * | 1-19 | TECHNICAL FIELDS SEARCHED (IPC) |

The present search report has been drawn up for all claims

| Place of search | Date of completion of the search | Examiner |
|---|---|---|
| The Hague | 28 November 2022 | Zalfen, Alina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 4 119 126 A1

## ANNEX TO THE EUROPEAN SEARCH REPORT
## ON EUROPEAN PATENT APPLICATION NO.

EP 22 18 3842

28-11-2022

| Patent document cited in search report | | Publication date | Patent family member(s) | | Publication date |
|---|---|---|---|---|---|
| US 2018185282 | A1 | 05-07-2018 | CL 2019001806 | A1 | 06-12-2019 |
| | | | EP 3565537 | A2 | 13-11-2019 |
| | | | US 2018185282 | A1 | 05-07-2018 |
| | | | WO 2018122598 | A2 | 05-07-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 201414585 A1
- WO 2007110422 A2
- WO 2015175573 A1
- WO 2010010431 A

### Non-patent literature cited in the description

- **LEELA SRINIVAS ; V. EC. SHALINI ; M. SHYLAJA, TURMERIN.** A Water Soluble Antioxidant Peptide from Turmeric [Curcuma longa. *Archives of Biochemistry and Biophysics,* 1992, vol. 292 (2), 617-623
- **PREETHA ANAND ; AJAIKUMAR B. KUNNUMAKKARA ; ROBERT A. NEWMAN ; BHARAT B. AGGARWAL.** Bioavailability of Curcumin: Problems and Promises. *Mol. Pharmaceutics,* 2007, vol. 4 (6), 807-818
- **G. SHOBA ; D. JOY ; T. JOSEPH ; M. MAJEED ; R. RAJENDRAN ; P. S. SRINIVAS.** Influence of piperine on the pharmacokinetics of curcumin in animals and human volunteers. *Planta medica,* 1998, vol. 64, 353-6
- **ALEX E. GRILL ; BRENDA KONIAR ; JAYANTH PANYAM.** Co-delivery of natural metabolic inhibitors in a self-microemulsifying drug delivery system for improved oral bioavailability of curcumin. *Drug Deliv. and Trans1. Res.,* 07 May 2014
- **MARIA ARTIGA-ARTIGAS ; YAMEL LANJARI-PEREZ ; OLGA MARTÍN-BELLOSO.** Curcumin-loaded nanoemulsions stability as affected by the nature and concentration of surfactant. *Food Chemistry,* 2018, vol. 266, 466-474
- **GUDDADARANGAVVANAHALLY K JAYAPRAKASHA ; KOTAMBALLI N CHIDAMBARA MURTHY ; BHIMANAGOUDA S PATIL.** Enhanced colon cancer chemoprevention of curcumin by nanoencapsulation with whey protein. *European Journal of Pharmacology,* 15 October 2016, vol. 789, 291-300
- **MING LI ; YING MA ; JIE CUI.** Whey-protein-stabilized Nanoemulsions as a Potential Delivery System for Water-insoluble Curcumin. *Food Science and Technology,* 2014, 1-10
- **ASGHAR ; KHUSHNUMA ; MOHD QASIM ; GANGAPPA DHARMAPURI ; DIBAKAR DAS.** Investigation on a smart nanocarrier with a mesoporous magnetic core and thermo-responsive shell for co-delivery of doxorubicin and curcumin: a new approach towards combination therapy of cancer. *RSC advances,* 2017, vol. 7 (46), 28802-28818
- **REZAEI ; ATEFE ; ALI NASIRPOU.** Evaluation of release kinetics and mechanisms of curcumin and curcumin-β-cyclodextrin inclusion complex incorporated in electrospun almond gum/PVA nanofibers in simulated saliva and simulated gastrointestinal conditions. *BioNanoScience,* 2019, vol. 9 (2), 438-445